# EUROPEAN PATENT APPLICATION

(11) **EP 1 894 577 A1**
(43) Date of publication of application: **05.03.2008**
(21) Application number: 06766486.2
(22) Date of filing: 08.06.2006
(51) Int. Cl.: A61K 51/00, G01T 1/161

(54) **RADIOACTIVE DIAGNOSTIC IMAGING AGENT**

(30) Priority: 14.06.2005 JP 2005173800
(71) Applicant: NIHON MEDI-PHYSICS CO., LTD., Nishinomiya-shi, Hyogo 662-0918 (JP)
(72) Inventor: SHIMADA, Takayuki, Sodegaura-shi Chiba 2990266 (JP); NAKATANI, Akira, Sodegaura-shi Chiba 2990266 (JP); TAKAHASHI, Keietsu, Sodegaura-shi Chiba 2990266 (JP)
(74) Representative: Wilhelms . Kilian & Partner Patentanwälte
(86) International application number: PCT/JP2006/311513
(87) International publication number: WO 2006/134822

(57) **Abstract**

It is intended to provide a radioactive diagnostic imaging agent comprising a radioactive halogen-labeled compound as an active ingredient, in which the active ingredient is prevented from radiolysis and its stability is improved. This is achieved by adding a biologically-acceptable sugar or sugar alcohol to the radioactive diagnostic imaging agent in an amount effective to prevent radiolysis. The amount of the sugar or sugar alcohol to be added is preferably 10 (mmol/L)/GBq/mL or more, and more preferably 50 (mmol/L)/GBq/mL or more. The sugar is preferably selected from the group consisting of erythrose, threose, ribose, arabinose, xylose, lyxose, allose, altrose, glucose, mannose, gulose, idose, galactose, talose, erythrulose, ribulose, xylulose, psicose, fructose, sorbose, and tagatose. The sugar alcohol is preferably selected from the group consisting of erythritol, xylitol, sorbitol, and mannitol.

## Description

### TECHNICAL FIELD

The present invention relates to a radioactive diagnostic imaging agent that contains a radioactive halogen-labeled compound as an active ingredient. More specifically, the present invention relates to a radioactive diagnostic imaging agent which is prevented from radiolysis of a radioactive halogen-labeled organic compound by addition of a sugar or a sugar alcohol.

### BACKGROUND ART

Nuclear medicine examination represented by positron emission tomography (hereinafter referred to as "PET") and single photon emission computed tomography (hereinafter referred to as "SPECT"), is effective in diagnosing a variety of diseases including cancer. These techniques involve administering an agent labeled with a specific radioisotope (hereinafter referred to as "radiopharmaceutical") to a patient, followed by detecting γ-ray emitted directly or indirectly from the administered agent. Nuclear medicine examinations is characteristic in terms of not only high specificity and sensitivity to diseases, but also in an advantage of providing information on the functioning of lesions, compared to other examination techniques.

For example, 2-[¹⁸F] fluoro-2-deoxy-D-glucose (hereinafter referred to as "¹⁸F-FDG"), one of radiopharmaceuticals used for PET examination, has a property of accumulating in an area where glucose metabolism is enhanced, thereby making it possible to specifically detect tumors in which glucose metabolism is enhanced.

Of the above nuclear medicine examination modalities, PET can provide high quality imaging that enables more effective diagnosis than SPECT that has clinically been used widely. PET is thus expected to offer a new diagnostic modality that follows SPECT, and radiopharmaceuticals for PET have been developed by many laboratories and the like. For example, various receptor mapping agents and bloodstream diagnostic agents have been synthesized and have been studied for clinical application.

A problem with the radiopharmaceuticals is that these agents tend to undergo radiolysis and their purity is gradually decreased. The decrease of purity due to the radiolysis is particularly serious for PET agents since radioactive nuclear species used in PET generally have greater radiation energy than the nuclear species used in SPECT.

Under the circumstances, various techniques have been investigated to protect radiopharmaceuticals against the effect of radiolysis.

International Publication Pamphlet No. WO03/090789 discloses a method in which radiolysis of ¹⁸F-FDG is prevented by addition of a weak acid buffer to a solution of ¹⁸F-FDG, as well as an injection prepared by this method (Patent Document 1). Also, International Publication Pamphlet No. WO04/043497 discloses an injection composition comprising a ¹⁸F-FDG solution to which ethanol is added to prevent the radiolysis of ¹⁸F-FDG and improve the stability of the composition (Patent Document 2).

Japanese Patent Laid-Open No. Hei 10-147542 discloses that an organic compound which has a reaction rate constant with OH radicals, H radicals or hydrated electron of 1 x 10⁸ to 5 x 10¹⁰ mol⁻¹s⁻¹ is used to protect radiopharmaceuticals against radiolysis (Patent Document 3). Sara Goldstein et al. reported that mannitol acts as a scavenger of OH radials in an aqueous solution (Non-Patent Document 1). Furthermore, Heli Teerijoki et al. and A. N. Ouraishi et al. reported that mannitol does not serve as a substrate for glucose transporters. In particular, A. N. Ouraishi et al. compared mannitol with 2-deoxyglucose for permeability to the human cell membrane (Non-Patent Documents 2 and 3).
Patent Document 1: International Publication Pamphlet No. WO03/090789
Patent Document 2: International Publication Pamphlet No. WO04/043497
Patent Document 3: Japanese Patent Laid-Open No. Hei 10-147542 Non-Patent Document 1: Sara Goldstein et al., Int. J. Radiat. Biol., 46, 6(1984):725-729
Non-Patent Document. 2: Heli Teerijoki et al., Comparative Biochemistry and Physiology Part B, 128 (2001) :483-491
Non-Patent Document 3: A. N. Ouraishi et al., Placenta, 20 (1999):167-174

### DISCLOSURE OF THE INVENTION

### PROBLEMS TO BE SOLVED BY THE INVENTION

As described above, different techniques have been proposed to protect ¹⁸F-FDG and other radiopharmaceuticals against the effect of radiolysis and improve the stability of radiopharmaceuticals.

However, the approach based on the addition of a weak acid buffer described in International Publication Pamphlet No. WO03/090789 causes a decrease in pH of the injection preparation. While quality of pharmaceuticals is required to be strictly controlled, buffers are generally composed of several components, and thus it requires complicated processes or is generally difficult to analyze the weak acid buffer and its lysates present in the preparation. Accordingly, it is desirable to use a stabilizing agent which is composed of a single-component and can be readily analyzed.

The approach described in International Publication Pamphlet No. WO04/043497 involves the addition of ethanol which is a single-component agent and can readily be analyzed advantageously. However, ethanol is regulated by a guideline for residual solvents and should be used in minimal amounts.

The radiation-shielding agent disclosed in Japanese Patent Laid-Open No. Hei 10-147542 can be selected from neutral organic compounds, and thus can protect the active ingredient of the radioisotope-labeled organic compound against radiolysis without decreasing the pH. However, the publication is silent about optimum conditions for radiopharmaceuticals that contain radioactive halogen-labeled organic compounds as active ingredients.

As stated above, there is a literature reporting that mannitol serves as an OH scavenger. However, use of mannitol as a stabilizing agent for ¹⁸F-FDG and other radioactive halogen-labeled organic compounds has not been studied yet.

The present invention has been devised in view of such circumstances, and it is an object of the present invention to provide an injection composition of a radiopharmaceutical that uses as an active ingredient a radioactive halogen-labeled organic compound including a radioactive fluorine-labeled organic compound, in which the active ingredient is prevented from radiolysis and its stability is improved.

### MEANS FOR SOLVING THE PROBLEMS

As a result of researches, the present inventors have found that the radiolysis of the active ingredient can be prevented by adding a sugar or a sugar alcohol to the above-mentioned radiopharmaceutical, and thus have completed the invention. Thus, the present invention provides a radioactive diagnostic imaging agent which comprises a radioactive halogen-labeled compound as an active ingredient, to which a safe or biologically-acceptable sugar or sugar alcohol is added in an amount effective to prevent radiolysis.

The radioactive halogen-labeled compound typically includes, but is not limited to organic compounds labeled with radioactive halogens. Examples thereof include 2-fluoro-2-deoxy-D-glucose (FDG) and various other glucose derivatives labeled with radioactive halogens, amino acid derivatives labeled with radioactive halogens, and cocaine derivatives labeled with radioactive halogens.

The radioactive halogen is not limited to a specific one, and may be various radioactive halogens that have been used in radioactive diagnostic imaging agents for PET, SPECT or the like, including ¹⁸F, ³⁴Cl, ⁷⁵Br, ⁷⁶Br, ⁸²Br, ⁸⁰Br, ¹²³I, and ¹²⁴I.

The sugar is not limited to a specific one as long as it has water-solubility. The sugar is preferably a neutral sugar, more preferably a monosaccharide or oligosaccharide, and still more preferably an aldose or ketose. The monosaccharide is preferably selected from the group consisting of triose, tetrose, pentose, and hexose, more preferably from the group consisting of erythrose, threose, ribose, arabinose, xylose, lyxose, allose, altrose, glucose, mannose, gulose, idose, galactose, talose, erythrulose, ribulose, xylulose, psicose, fructose, sorbose, and tagatose, and particularly preferably from the group consisting of erythrose, threose, ribose, xylose, lyxose, allose, altrose, gulose, idose, talose, erythrulose, ribulose, xylulose, psicose, sorbose, and tagatose.

The sugar alcohol is not limited to a specific one as long as it has water-solubility. The sugar alcohol is preferably selected from the group consisting of tritol, tetritol, pentitol, and hexitol, more preferably from the group consisting of erythritol, xylitol, sorbitol, and mannitol, and particularly preferably from the group consisting of erythritol, xylitol, and mannitol.

The amount of sugar or sugar alcohol to be used is not limited to specific one as long as it is not less than an amount effective to prevent radiolysis. It is preferably 10 (mmol/L)/GBq/mL or more, more preferably 50 (mmol/L)/GBq/mL or more, and most preferably 100 (mmol/L)/GBq/mL or more at the time of certification. The unit (mmol/L)/GBq/mL is defined as the molar concentration per 1 GBq/mL of radioactive concentration. Thus, the above-indicated addition amounts are equivalent to approximately 1 mmol/L, approximately 5 mmol/L and approximately 10 mmol/L respectively, when they are converted to addition amounts in a preparation having a radioactive concentration of 92.5 MBq/mL. More specifically, the addition amount is preferably 2 µmol or more, more preferably 10 µmol or more, and most preferably 20 µmol or more, when the radioactivity is 185 MBq at the time of certification and the volume of the preparation is 2 mL. When the amount of the sugar or sugar alcohol is too small, prevention of radiolysis is not achieved sufficiently, and thus this is not preferred.

The term "at the time of certification" is defined as the date and time when the radioactivity indicated in a product, namely, the radioactivity specified by the standard is exhibited. For example, if a product has the indicated radioactivity of 185 MBq and indicates the date and time of certification to be at 1 p.m. on June 4th, the product is prepared with a radioactivity being adjusted to meet 185 MBq of the radioactivity specified in the standard at 1 p.m. on June 4th of the time of certification.

In the meantime, the amount of the sugar or sugar alcohol must be adjusted within a range that is acceptable for additives to injections. This range is determined considering, for example, an acceptable daily dose of each additive. For example, maximum doses for intravenous injection of typical sugars or sugar alcohols found in literature are as follows: mannitol = 1.2 g; xylitol = 200 mg; sorbitol = 1.5 g; glucose = 8 g; fructose = 900 mg; maltose = 10 g; and lactose = 1250 mg ("Pharmaceutical Additives Directory 2000", published by Yakuji Nippo, Ltd., edited by The Japan Pharmaceutical Additives Association (2000)). When these sugars or sugar alcohols are used as additives, the addition amounts thereof should be determined in such a way that their ultimate doses administered with the injection do not exceed the maximum doses.

The type of the sugar or sugar alcohol to be used is selected depending on the type and in vivo kinetics of the radioactive halogen-labeled compound used as the active ingredient. More specifically, the sugar or sugar alcohol is preferably one that is considered not to impede the expression of efficacy of active ingredients considering in vivo kinetics thereof. For example, when the active ingredient is ¹⁸F-FDG, the sugar or sugar alcohol is selected from compounds that do not serve as a substrate for glucose transporters, and is preferably selected from the group consisting of fructose, ribose, sucrose, mannitol, xylitol, and sorbitol, and is most preferably mannitol.

### EFFECTS OF THE INVENTION

The radioactive diagnostic imaging agent of the present invention prevents radiolysis of radioactive fluorine-labeled compounds and other radioactive halogen-labeled compounds that serve as the active ingredient, and thus is inhibited from decrease in purity during transportation or storage.

### BEST MODE FOR CARRYING OUT THE INVENTION

Hereinafter, a process for producing the radioactive diagnostic imaging agent according to the present invention will be described.

In the production of the radioactive diagnostic imaging agent of the present invention, a radioactive halogen-labeled compound as the active ingredient is first synthesized. Different radioactive halogen-labeled compounds can be synthesized using known techniques developed for each compound. For example, when the radioactive halogen-labeled compound is ¹⁸F-FDG, it can be synthesized with a technique devised by Hamacher et al. (K. Hamacher et al., Applied Radiation and Isotopes, (Great Britain), Pergamon Press, 41, 1(1990):49-55) (hereinafter referred to as "Hamacher method").

Now, the process for producing the radioactive diagnostic imaging agent of the present invention will be explained with reference to an example in which the radioactive halogen-labeled compound that serves as the active ingredient is ¹⁸F-FDG.

According to the Hamacher method, target water (¹⁸O-enriched water) is first exposed to proton bombardment to obtain [¹⁸F] fluoride ions in the form of the target water containing [¹⁸F] fluoride ions, prior to the synthesis of ¹⁸F-FDG. The target water containing [¹⁸F] fluoride ions is then passed through a column packed with anion exchange resin to adsorb and collect [¹⁸F] fluoride ions onto the resin. An aqueous potassium carbonate solution is then passed through the column to elute the [¹⁸F]fluoride ions that have been colleted onto the resin. The eluate is collected in a reaction vessel.

Next, [¹⁸F]fluoride ions are activated by adding a solution in acetonitrile of aminopolyether as a phase transfer catalyst to the eluate, and evaporating the mixture to dryness. To the resulting residue, a solution in acetonitrile of 1,3,4,6-tetra-O-acetyl-2-O-trifluoromethanesulfonyl-β-D-mannopyranose (hereinafter referred to as "TATM") is added, and the mixture is heated (for example, at 85°C for 5 min) for nucleophilic substitution reaction to synthesize 1,3,4,6-tetra-O-acetyl-2-fluoro-2-deoxyglucose (hereinafter referred to as "TAFDG"). The reaction mixture is then dried to substantially remove the organic solvent, followed by addition of hydrochloric acid and heating (for example, at 120°C for 15 min) for deprotection. This gives [¹⁸F]-FDG. The resulting [¹⁸F] -FDG is purified and supplemented with physiological saline or the like to obtain ¹⁸F-FDG with a desired radioactive concentration.

The radioactive fluorine-labeled compound obtained in the above-described manner is then mixed with a required amount of a sugar or a sugar alcohol to obtain the radioactive diagnostic imaging agent of the present invention. The timing of mixing is not limited to a specific one as long as the final preparation contains the required amount of the sugar or sugar alcohol. For example, a high-concentration solution of ¹⁸F-FDG and a high-concentration solution of sugar or sugar alcohol are prepared in advance and are mixed together in an appropriate proportion so that ¹⁸F-FDG and the sugar or sugar alcohol are present in the final preparation at their respective desired concentrations. Specifically, when it is desired to prepare a 700 MBq ¹⁸F-FDG solution containing 15 mmol/L mannitol, a 1.4 GBq ¹⁸F-FDG solution is mixed with an equal volume of a 30 mmol/L mannitol solution.

### Examples

The present invention will now be described in further detail with reference to Test Examples, Examples, and Comparative Examples, which are not intended to limit the scope of the invention in any way.

### Examples 1 through 12, and Comparative Example 1

An ¹⁸F-FDG solution was prepared in accordance with the following procedure.

First, ¹⁸O-enriched target water was exposed to proton bombardment to obtain [¹⁸F]fluoride ions in the form of the target water containing [¹⁸F]fluoride ions. The target water was then passed through strong anion exchange resins to adsorb and collect [¹⁸F]fluoride ions onto the resins. An aqueous potassium carbonate solution was then passed therethrough to elute the [¹⁸F]fluoride ions.

To the eluate containing [¹⁸F] fluoride ions, a solution in acetonitrile of KRYPTOFIX 222 (under trade name, manufactured by Merck & Co., Inc.) was added, and the mixture was heated and evaporated to dryness. Then, a solution of TATM in acetonitrile was added thereto, and the mixture was heated for ¹⁸F-labeling. Subsequently, the product was hydrolyzed by hydrochloric acid for deprotection. The resulting mixture was purified by use of an ODS column and an alumina column to obtain ¹⁸F-FDG stock solution (radioactivity = 104.3 GBq). The ¹⁸F-FDG stock solution was diluted with physiological saline so that the resulting solution had 92.5 MBq/mL 4.5 hours after preparation. This gave a ¹⁸F-FDG solution.

To 2 mL of the above-obtained ¹⁸F-FDG solution, the solutions of sugars and sugar alcohols having concentrations shown in Table 1 were each added in amounts shown in Table 1, and mixed at room temperature to prepare respective sample solutions.

**Table 1**

| Types of added sugars or sugar alcohols, concentrations and addition amounts of employed sugars or sugar alcohols, and concentrations of sugars or sugar alcohols in the resultant sample solutions | | | | |
|---|---|---|---|---|
| | Sugars or sugar alcohols added | Conc. of solutions of sugars or sugar alcohols employed (mmol/L) | Addition amounts of solutions of sugars or sugar alcohols (mL) | Conc. of sugars or sugar alcohols in the resultant sample solutions (mmol/L) |
| Comparative Example 1 | - | - | 0 | 0 |
| Example 1 | Glucose | 28 | 0.07 | 0.98 |
| Example 2 | | 139 | 0.07 | 4.9 |
| Example 3 | | 278 | 0.07 | 9.7 |
| Example 4 | Fructose | 28 | 0.07 | 0.98 |
| Example 5 | | 139 | 0.07 | 4.9 |
| Example 6 | | 278 | 0.07 | 9.7 |
| Example 7 | Xylitol | 33 | 0.06 | 0.99 |
| Example 8 | | 164 | 0.06 | 4.9 |
| Example 9 | | 329 | 0.06 | 9.9 |
| Example 10 | Mannitol | 41 | 0.05 | 1.0 |
| Example 11 | | 206 | 0.05 | 5.2 |
| Example 12 | | 412 | 0.05 | 10 |

The above-obtained samples were subjected to TLC analysis on the below conditions immediately after the preparation of ¹⁸F-FDG solution (Comparative Example 1 only), and 4.5 and 8.5 hours after the preparation. Radiochemical purity was obtained based on the area percentage of the ¹⁸F-FDG peak. The radiochemical purity at each time point was compared to one another to evaluate stability of the samples.

### TLC conditions:

TLC plate = Silica Gel 60 F₂₅₄ (under trade name, manufactured by Merck & Co., Inc.)
Developing solvent = acetonitrile/water = 19:1
Developing length = 10 cm
Detector = Rita Star (under trade name, manufactured by Raytest)

The results are shown in Table 2.

As can be seen from Table 2, each of the ¹⁸F-FDG solutions containing sugars or sugar alcohols (Examples 1 through 12) showed a higher radiochemical purity as compared to the sugar- or sugar alcohol-free ¹⁸F-FDG solution (Comparative Example 1) 4.5 and 8.5 hours after preparation, indicating significant suppression of radiolysis.

These results indicate that each of the sugars or sugar alcohols used in Examples 1 through 12 can significantly prevent the decrease in the radiochemical purity of ¹⁸F-FDG caused by radiolysis.

**Table 2**

| Quantified radiochemical purity of each sample at different time points | | | |
|---|---|---|---|
| | Radiochemical purity (%) | | |
| | Immediately after preparation | 4.5 hours after preparation | 8.5 hours after preparation |
| Comparative Example 1 | 95.0 | 87.0 | 86.2 |
| Example 1 | - | 92.2 | 91.6 |
| Example 2 | - | 93.2 | 93.3 |
| Example 3 | - | 93.5 | 93.5 |
| Example 4 | - | 91.4 | 90.8 |
| Example 5 | - | 93.1 | 92.6 |
| Example 6 | - | 93.4 | 93.2 |
| Example 7 | - | 92.0 | 91.6 |
| Example 8 | - | 93.9 | 93.1 |
| Example 9 | - | 94.2 | 94.0 |
| Example 10 | - | 92.1 | 91.6 |
| Example 11 | - | 93.4 | 92.9 |
| Example 12 | - | 93.8 | 93.5 |

### Examples 13 through 18, and Comparative Example 2

The same procedure as in Comparative Example 1 and Examples 1 through 12 was repeated to obtain a ¹⁸F-FDG stock solution (radioactivity = 126.3 GBq). To the ¹⁸F-FDG stock solution, physiological saline was added for dilution so that the resulting solution had 92.5 MBq/mL 4.5 hours after preparation. This gave a ¹⁸F-FDG solution.

To 2 mL of the above-obtained ¹⁸F-FDG solution, the mannitol solutions having different concentrations shown in Table 3 were each added in amounts shown in Table 3, and mixed at room temperature to prepare respective sample solutions.

**Table 3**

| Addition amounts of mannitol solution and physiological saline used in samples of Examples 13-18 and concentrations of mannitol in the resultant samples | | | |
|---|---|---|---|
| | Conc. of mannitol solution employed (mmol/L) | Addition amounts of mannitol solution (mL) | Conc. of mannitol solution (mmol/L) in the resultant samples |
| Comparative Example 2 | - | 0 | 0 |
| Example 13 | 165 | 0.03 | 2.5 |
| Example 14 | 165 | 0.06 | 5.0 |
| Example 15 | 494 | 0.03 | 7.4 |
| Example 16 | 494 | 0.04 | 9.9 |
| Example 17 | 494 | 0.06 | 15 |
| Example 18 | 823 | 0.06 | 25 |

The above-obtained samples were subjected to TLC analysis on the below conditions immediately after the preparation of ¹⁸F-FDG solution (Comparative Example 2 only), and 4.5 and 8.5 hours after the preparation. Radiochemical purity was obtained based on the area percentage of the ¹⁸F-FDG peak. The radiochemical purity at each time point was compared to one another to evaluate stability of the samples.

### TLC conditions:

TLC plate = Silica Gel 60 F₂₅₄ (under trade name, manufactured by Merck & Co., Inc.)
Developing solvent = acetonitrile/water = 19:1
Developing length = 10 cm
Detector = Radiochromanizer (JTC-R75-21361, manufactured by Aloka)

The results are shown in Table 4.

As can be seen from Table 4, each of the samples containing mannitol (Examples 13 through 18) showed a significantly higher radiochemical purity as compared to the mannitol-free sample (Comparative Example 2) 4.5 and 8.5 hours after preparation.

These results indicate that mannitol can significantly prevent the decrease in the radiochemical purity of ¹⁸F-FDG caused by radiolysis when added at 2.5 mmol/L or higher concentrations.

**Table 4**

| Quantified radiochemical purity of each sample at different time points | | | |
|---|---|---|---|
| | Radiochemical purity (%) | | |
| | Immediately after preparation of FDG solution | 4.5 hours after preparation | 8.5 hours after preparation |
| Comparative Example 2 | 96.0 | 87.5 | 87.4 |
| Example 13 | - | 94.6 | 94.6 |
| Example 14 | - | 94.9 | 94.6 |
| Example 15 | - | 95.2 | 95.0 |
| Example 16 | - | 95.5 | 95.2 |
| Example 17 | - | 95.5 | 95.2 |
| Example 18 | - | 95.6 | 95.6 |

### INDUSTRIAL APPLICABILITY

The present invention is useful for preventing radiolysis of radiopharmaceuticals that contain radioactive halogen-labeled compounds as active ingredients, and thus can be utilized in the field of nuclear medicine.

## Claims

1. A radioactive diagnostic imaging agent which comprises a radioactive halogen-labeled compound as an active ingredient, to which a biologically-acceptable sugar or sugar alcohol is added in an amount effective to prevent radiolysis.

2. The radioactive diagnostic imaging agent according to claim 1, wherein the radioactive halogen is selected from the group consisting of ¹⁸F, ³⁴Cl, ⁷⁵Br, ⁷⁶Br, ⁸²Br, ⁸⁰Br, ¹²³I, and ¹²⁴I.

3. The radioactive diagnostic imaging agent according to claim 1 or 2, wherein the sugar or the sugar alcohol is present in an amount of 10 (mmol/L)/GBq/mL or more.

4. The radioactive diagnostic imaging agent according to claim 3, wherein the sugar or the sugar alcohol is present in an amount of 50 (mmol/L)/GBq/mL or more.

5. The radioactive diagnostic imaging agent according to claim 1 or 2, wherein the sugar or the sugar alcohol is present at a concentration of 1 mmol/L or higher in a 92.5 MBq/mL preparation.

6. The radioactive diagnostic imaging agent according to claim 1 or 2, wherein the sugar or the sugar alcohol is present at a concentration of 5 mmol/L or higher in a 92.5 MBq/mL preparation.

7. The radioactive diagnostic imaging agent according to any one of claims 1 to 6, wherein the sugar is a neutral sugar.

8. The radioactive diagnostic imaging agent according to claim 7, wherein the sugar is a monosaccharide or oligosaccharide.

9. The radioactive diagnostic imaging agent according to claim 8, wherein the sugar is an aldose or ketose.

10. The radioactive diagnostic imaging agent according to claim 8 or 9, wherein the sugar is selected from the group consisting of triose, tetrose, pentose, and hexose.

11. The radioactive diagnostic imaging agent according to claim 10, wherein the sugar is selected from the group consisting of erythrose, threose, ribose, arabinose, xylose, lyxose, allose, altrose, glucose, mannose, gulose, idose, galactose, talose, erythrulose, ribulose, xylulose, psicose, fructose, sorbose, and tagatose.

12. The radioactive diagnostic imaging agent according to claim 10, wherein the sugar is selected from the group consisting of erythrose, threose, ribose, xylose, lyxose, allose, altrose, gulose, idose, talose, erythrulose, ribulose, xylulose, psicose, sorbose, and tagatose.

13. The radioactive diagnostic imaging agent according to any one of claims 1 to 6, wherein the sugar alcohol is selected from the group consisting of tritol, tetritol, pentitol, and hexitol.

14. The radioactive diagnostic imaging agent according to claim 13, wherein the sugar alcohol is selected from the group consisting of erythritol, xylitol, sorbitol, and mannitol.

15. The radioactive diagnostic imaging agent according to claim 13, wherein the sugar alcohol is selected from the group consisting of erythritol, xylitol, and mannitol.
